# EUROPEAN PATENT APPLICATION

(11) **EP 2 610 238 A1**
(43) Date of publication of application: **03.07.2013**
(21) Application number: 11870840.3
(22) Date of filing: 18.08.2011
(51) Int. Cl.: C07C 59/08, C07C 51/16

(54) **OXIDATIVE CATALYTIC PROCESS FOR THE SYNTHESIS OF LACTIC ACID**

(30) Priority: 18.08.2010 BR PI1004306
(71) Applicant: Petroleo Brasileiro S.A. - PETROBRAS, CEP 20035-900-Rio de Janeiro (BR); Instituto Nacional De Tecnologia - INT, Cep: 20081-312 Rio de Janeiro - RJ (BR)
(72) Inventor: FRAGA, Marco André, CEP: 22221-070 Rio de Janeiro - RJ Brasil (BR); DE ALBUQUERQUE, Elise Mota, CEP: 20720-350 Rio de Janeiro - RJ Brasil (BR); CANDIDO, Robert Amaral, CEP: 24230-050 Niterói - RJ Brasil (BR)
(74) Representative: Benson, John Everett
(86) International application number: PCT/BR2011/000290
(87) International publication number: WO 2013/023257

(57) **Abstract**

Current industrial processes for the production of lactic acid are fermentative, using lactic bacteria which require large volumes and create large quantities of liquid residue which need to be treated. Chemical literature also describes the use of reaction systems with homogeneous catalysis, which also pose problems which in turn impact our costs, owing to the increased requirements in terms of the control of the process and the type of reactor necessary. Therefore, the process used thus far, carried out in hydrogenolysis, isomerisation and oxidation are defective, owing to the significant formation of sub-products, mainly pyruvic acid and acetic acid and the low yield of lactic acid.

Lactic acid can also be obtained by the chemical transformation of other sources other than starch, but which are also renewable. The present invention provides a oxidative process for the production of lactic acid, in which the reaction with pure oxygen or oxygen mixed with air takes place below 100°C and under autogenous pressure, using a noble metal catalyst in a metal oxide. The process uses 1,2-propanediol as primary material, derived from the reaction of hydrogenolysis of the glycerine, and reaches yields of more than 70% of lactic acid and less than 30% in sub-products, pyruvic acid and acetol.

## Description

### FIELD OF THE INVENTION

The present invention relates to a process for obtaining lactic acid for the selective oxidation of 1,2-propanediol. The present invention describes an oxidative process with yields of more than 70%, for the production of lactic acid from 1,2-propanediol, in an an alkaline medium, at a low temperature and under atmospheric or autogenous pressure, using a noble metal catalyst supported on metal oxide.

### BACKGROUND OF THE INVENTION

Lactic acid is presented as one of the important inputs for the petrochemical industry, since as well as being used to obtain biodegradable materials it is synthesised from renewable sources, like corn glucose, molasses and cheese whey.

Current industrial processes are fermentative, using lactic bacteria. These micro-organisms used in the process have complex requirements in terms of growth, requiring vitamins and amino acids for their cultivation. Moreover, the fermentative processes are time-consuming, requiring large volumes and creating large quantities of liquid residue which need to be treated.

Lactic acid can also be obtained by the chemical transformation of other sources other than starch, but which are also renewable. In this sense, some proposals use glycerine in one process wherein the reaction takes place in an alkaline medium, in a homogeneous phase and under hydrothermal conditions. Although the yields of lactic acid reach around 90%, the reactions take place at very high temperatures (300°C) and fairly high pressures. These conditions lead to expensive processes, since, as well as the high energy consumption, they require equipment which is made with special materials, in order to avoid corrosion of the reactors resulting from the high concentration of hydroxide.

The EP 2100871 document describes the use of organic composts with three carbon atoms, being formed by a primary alcohol or an aldehyde which contains a hydroxyl group in the alpha position in relation to the hydroxyl of the primary alcohol or to the carbonyl of the aldehyde, as the raw material. Using this type of input, which includes 1,2-propanediol, the catalytic process is based on a hydrogenolysis reaction taking place, therefore, in the presence of hydrogen and the use of temperatures in the order of 90°C to 170°C being necessary. During the process even more hydrogen is produced, and it is essential to prevent it from reacting with the oxygen in the air. To this end, the document makes it clear that it is necessary to control the atmosphere of the reactor, additional stages being indicated for the purging of the system with nitrogen, meaning that controlling the process is more complex. Moreover, the formation of sub-products is noted, such as acetic acid and superior aliphatic acids.

Similar hydrogenolysis processes to the one described above are revealed in scientific literature (E.P. Maris, W.C. Ketchie, M. Murayama, R.J. Davis, J. Catal. 215 (2007) 281-294andE.P. Maris, R.J. Davis, J. Catal. 249 (2007) 328-337), except, coming directly from glycerine. Although the ethylene glycol and the propylene glycol are the main products of this reaction, the authors will identified the possibility of producing lactic acid by means of the addition of alkali metal hydroxides in the presence of ruthenium or platinum catalysts, or even bimetallic systems of these metals with gold. However, the yields are moderate or low, covering a range between 8.5% and 45%.

Recently, isomerisation reactions using oxidated derivates of glycerine, essentially dihydroxyacetone and glycolaldehyde, have been proposed for the synthesis of lactic acid (R.M. West, M.S. Holm, S. Saravanaurugan, J. Xiong, Z. Beversdorf, E. Taarning, C.H. Christensen, J. Catal. 269 (2010) 122-130). In these cases, the reaction takes place in the presence of an acid catalyst with a zeolitic structure, preferably H-USY zeolite, between 115°C and 125°C and under autogenous pressure. The yields reach up to around 70% with some zeolites, however, the high pressures used during the reaction must be highlighted.

Oxidative methods using heterogeneous catalysts have also been revealed, coming from glycerine as well as 1,2-propanediol. The patent document CN 101225041 (L. Haichao, S. Yihong, L. Hongjia, CN101225041 A, Jul. 23, 2008) describes a process wherein it is possible to obtain lactic acid, but, it has much lower yields in the conditions specified in said document, varying in the range between 9.7% and 32% and reaching, at maximum, 81% of glycerine conversion.

There are works in scientific literature which approach the subject of the oxidation of diols and essentially focus on the application of gold-based metallic catalysts, at temperatures in the order of 70° to 90°C and under a pressure of between 2 bar and 3 bar of pure oxygen.

The yields of lactic acid are always in the range of 5% to 64% and, eventually, this performance is the cause for comparison with platinum or palladium-based systems supported on activated carbon, owing to operational requirements (S. Demirel, P. Jern, M. Lucas, P. Claus, Catal. Today 122 (2007) 292-300; L. Prati, M. Rossi, J. Catal. 176 (1998) 552-560; C. Bianchi, F. Porta, L. Prati, M. Rossi, Top. Catal. 13 (2000) 231-236).

Ultimately, other studies deal with the production of pyruvic acid via oxiditave routes from 1,2-propanediol, which use platinum or palladium catalysts supported on activated carbon and promoters such as led, bismuth or tin (T. Tsujino, S. Oigashi, K. Kawashiro, H. Hayashi, J. Mol. Catal. 71 (1992) 25-35 and H.H.C.M. Pinxt, B.F.M. Kuster, G.B. Marin, Appl. Catal. A 191 (2000) 45-54).

The results of these works show that lactic acid is produced as a sub-product, using routes and processes that are not suitable for the synthesis of the lactic acid.

### DISTINCTION FROM THE PRIOR ART

In all of the processes described above, the production of lactic acid takes place from mixtures of glycerine or 1,2-propanediol, using different temperature conditions, total pressure and concentration of the components and the presence of a catalyst or not. Amongst the products, as well as lactic acid, other compounds are described. The literature also describes the use of reaction systems with a homogeneous catalyst, which also poses problems in terms of the separation and re-using of the catalyst in the process. Other references cite the use of pressures above atmosphere pressure, which also has an impact on our costs owing to larger requirements in terms of controlling the process and the type of reactor needed.

In general, the processes of the prior art were developed to promote the production of pyruvic acid. The low yield of lactic acid and the significant formation of sub-products, mainly pyruvic acid and acetic acid, necessarily require the use of additional stages, which allow the lactic acid to be purified. Naturally, the use of unitary separation operations leads to an increase of installation and operation costs.

The present invention, on the contrary, describes a selective catalytic process, with yields of more than 70% for the production of lactic acid.

### SUMMARY OF THE INVENTION

The invention relates to the creation of lactic acid with a high yield by means of the selective oxidation of 1,2-propanediol. The reaction takes place in the presence of oxygen and of an activated catalyst, which comprises a noble metal supported on metal oxide. The oxidation of the primary carbon which contains an OH group is selective at temperatures of less than 100°C, under atmospheric or autogenous pressure and in an alkaline medium. By means of these conditions, yields in the order of 70% of lactic acid are obtained, using equipment which is already installed and normally used in industrial chemical plants and with an energy consumption which is less than those practised in processes in the prior art. The catalyst is easily recovered by filtration at the end of the process.

### DETAILED DESCRIPTION OF THE INVENTION

The process presented in the present document enables lactic acid with yields of 70% or more to be obtained, by means of the use of heterogeneous catalysts which provide high selectivity and, also, high yields for lactic acid, using only oxygen in the air and 1,2-propanediol as reactants, at temperatures of less than 100° and under atmospheric pressure.

In certain embodiments of this invention it is possible to attain a complete conversion of 1,2-propanediol and only formation of lactic acid and, as a sub-product, pyruvic acid.

The manufacture process of lactic acid involves the use of a gaseous current selected from air, pure oxygen or a mixture of both, which is boiled in a reactor containing an aqeous solution of 1,2-propanediol at atmospheric pressure in a alkaline medium. This reactor also contains previously activated solid catalyst, in order to convert the reactants into lactic acid, preferably. This process can be carried out in a semi-continuous, continuous, semi-batch regime or a combination of these, either in a gaseous stage, or in a liquid phase.

In the process of this invention, the 1,2-propanediol is converted into lactic acid by means of the oxidation reaction of the primary carbon. The oxidation can also take place in the secondary carbon forming acetol. In higher temperatures, the acetol reacts with the oxygen of the gaseous current resulting in the formation of pyruvic acid. Therefore, the main sub-products of the obtainment of lactic acid with this process are acetol and pyruvic acid.

The process of the present invention comprises the following stages: 1^{st}) Activation of the catalyst: reduction of the catalyst at 350°C for 2 hours under an H₂ flow.
2^{nd}) Supplying the reactor: charging the reactor, equipped with a refux system, with a solution of 1,2-propanediol and with the pre-reduced catalyst.
3^{rd}) Reaction: actuation of heating, stirring and bubbling of oxygen or air maintaining the pH fixed with continuous dropping of an alkaline solution.
4^{th}) Separation of the products: removal of the catalyst by filatration and separation of the lactic acid of the aqueous phase.

Prepare the catalyst by wet or dry impregnation or by deposition-precipitation, with a solution of the metal precursor selected from hydroxides, nitrates, chlorides, sulphides, acetates and acetylacetonates or another compound which decomposes to form the corresponding metal oxide after calcination. The content of noble metal in the catalyst varies in a range between 0.01% and 10%, preferably between 0.1% and 5% p/p.

The support must have a particularly large surface area or large enough to guarantee a good dispersion of the metal, in the range between 50 m²g⁻¹ and 1000 m²g⁻¹ The support is selected from gamma-Al₂O₃, TiO₂, SiO₂ and ZrO₂, Nb₂O₅, CeO₂, MgO, ZSM-5, MCM-22, MCM-41, preferably Al₂O₃, TiO₂, SiO₂ and ZrO₂ The noble metal is selected from Pt, Pd, Ru, Rh and Ir, preferably Pt and Pd. In one embodiment of the invention, an oxidation catalyst is used which comprises one of the noble metals, or a combination of these, supported on a pure metal oxide, on a mixture of metal oxides or on aluminium silicates with a zeolitic structure. Preferably, the impregnation of the catalyst is carried out using a solution of hexachloroplatinic acid (H₂PtCl₆). The catalyst can also be obtained via dry impregnation. In this case, a compound selected from H₂Pt(OH)₆, Pt(NO₃)₄, Pt(NH₃)₄(NO₃)₂, Pt(NH₃)₄(OH)₂, PtCl₄, Pt(NH₄)₂Cl₄, Pt(NH₄)₂Cl₆, Pt(C₅H₇O₂)₂ or any other compound which decomposes to form PtO₂ is used as the platinum precursor. In another embodiment of this invention, a commercial catalyst of Pt/Al₂O₃, with 5% p/p of previously reduced Pt is used.

The reduction of the catalyst is carried out ex-situ at temperatures of between 200°C and 500°C or in-situ in the temperature range of 30°C to 100°C. In this case, the catalyst is added to the propanediol solution and is stirred continuously. The reduction can also be carried out sequentially, ex-situ or in-situ in the same temperature ranges described.

The oxidation reaction of the 1,2-propanediol is carried out in a reactor using 1,2-propanediol, in its pure form or in an aqueous solution, and a catalyst as reactants, in quantities which fulfil the catalyst/1,2-propanediol ratio in the range between 1/4 p/p and 1/20 p/p, keeping the pH of the reaction the same, at a selected value in the range betweem 7 and 14.0, preferably between 8 and 12, by means of the controlled addition of an alkaline solution, selected from the solutions of hydroxides and carbonates of alkaline metals and alkaline earth metals, preferably NaOH or KOH, with a concentration in the range between 0.1 M and 2 M, preferably in the range between 0.5 M and 1.5 M, at the selected temperature in the range between 30°C and 100°C, autogenous pressure between 1 bar and 5 bar, being stirred in the range between 200 rpm and 2000 rpm.

The input of oxygen into the reactor is carried out using air, pure oxygen or a mixture of oxygen-enriched air. The latter is obtained via membranes or any other suitable technology.

The conversion of 1,2-propanediol is completed after 5 hours of reaction, forming lactic acid in significant quantities, compared to the other products. The selectivity of the lactic acid remains at around 70% for the duration of the reaction period. Other detected products are pyruvic acid and acetol.

Using a preferred embodiment of the present invention, tests of performance appraisal of the catalysts were carried out in a device containing a glass reactor, a mechanical stirrer, a system for adding alkaline solution by means of a dosing pump connected to a pH measurer. The alkaline solution selected was NaOH with a concentration of 1 M. An aqueous solution of 1,2-propanediol with a concentration of 0.2 M, a flow rate of synthetic air (20% of O₂ in N₂ v/v) between 10 mLmin⁻¹ and 100 mLmin⁻¹ and a pH comprised between 7.0 and 14.0 and kept constant by means of the addition of an alkaline solution were used. The reaction temperature varied in the range comprised between 30°C and 80°C and the reaction pressure in the gap between 1 bar and 5 bar. Mechanical stirring was maintained between 500 rpm and 2000 rpm. Aliquots of solution were withdrawn every 30 mins and the products were analysed, by HPCL liquid chromatography, then filtration for the separation of the catalyst. The following examples describe the invention in a clear and sufficient manner, but are only illustrative and in no way limit the scope of protection of the present invention.

### EXAMPLES

### Example 1:

The platinum catalyst on alumina containing 5% p/p Pf was prepared by wet impregnation using a commercial alumina as a support and the precursor salt hexachloroplatinic acid. The first stage of preparation consisted of calcination of the support which was carried out in a muffle kiln from ambient temperature to 500°C following a heating rate of 10°Cmin⁻¹, maintained at 500°C for 4 hours. Then, the hexachloroplatinic acid was solubilised in water. This solution was added to the support (which was already calcinated) and this suspension was stirred for 1 hour at ambient temperature. After this stage, the vacuum of the material was dried at 80°C. Finally, the solid obtained was kept in an oven at 100°C for 12 hours and was, then, calcinated at 500°C for 4 hours with a heating rate of 10°C/min and synthetic air flow with a flow rate of 60 mLmin⁻¹.

### Example 2:

The platinum catalyst on alumina containing 5% p/p Pt prepared as described in Example 1 above was activated ex situ, that is, it was heated from ambient temperature to 350°C following a heating rate of 10°Cmin⁻¹, maintained at 350°C for 2 hours and using a pure hydrogen current at the rate of 50 mLmin⁻¹.

### Example 3:

The platinum catalyst on alumina containing 5% p/p Pt prepared as described in Example 1 and activated ex-situ as described in Example 2 was weighed, transferred to a 500 mL glass reactor containing 200 mL of distilled water and activated again, this time in situ, the reactor being heated at 90°C and a current of 50 mLmin⁻¹ of pure hydrogen being used, introduced into the suspension by a bubbler connected to the reactor. The suspension was stirred at 600 rpm and this condition was maintained for 1 hour. Evaporation of the water was avoided by using a reflux condenser with a water current in the serpentine.

### Example 4:

The platinum catalyst on alumina containing 5% p/p Pt prepared and activated according to Examples 1 to 3 was used in the oxidation reaction of 1,2-propanediol. An aqueous solution of 1,2-propanediol with a concentration of 0.2 M was added to the reactor containing 1 g of catalyst under stirring at 700 rpm. A glass electrode for measuring the pH of the reactional medium was connected to the reactor. Similarly, it was connected to a burette containing a 1 M solution of NaOH enabling it to be dropped in by manual action. The pH in the reactor was adjusted to 8.0 by means of the addition of a sufficient quantity of the NaOH solution and maintained for the duration of the reaction. A flow of 30 mLmin⁻¹ of air was let in through the bubbler. The reaction temperature of 40°C was maintained. After 5 hours of reaction in these conditions, a total conversion of 1,2 propanediol and the following distribution of selectivities between lactic acid, pyruvic acid and acetol were obtained: 65%, 23% and 12%, respectively.

### Example 5:

The platinum catalyst on alumina containing 5% p/p Pt prepared and activated according to Examples 1 to 3 was used in the oxidation reaction of 1,2-propanediol. An aqueous solution of 1,2-propanediol with a concentration of 0.2 M was added to the reactor containing 1 g of catalyst under stirring at 700 rpm. A glass electrode for measuring the pH of the reactional medium was connected to the reactor. Similarly, it was connected to a burette containing a 1 M solution of NaOH enabling it to be dropped in by manual action. The pH in the reactor was adjusted to 10.0 by means of the addition of a sufficient quantity of the NaOH solution and maintained for the duration of the reaction. A flow of 30 mLmin⁻¹ of air was let in through the bubbler. A reaction temperature of 40°C was maintained. After 6 hours of reaction in these conditions, a total conversion of 1,2 propanediol and the following distribution of selectivities between lactic acid, pyruvic acid and acetol were obtained: 70%, 19% and 11 %, respectively.

### Example 6:

The platinum catalyst on alumina containing 5% p/p Pt prepared and activated according to Examples 1 to 3 was used in the oxidation reaction of 1,2-propanediol. An aqueous solution of 1,2-propanediol with a concentration of 0.2 M was added to the reactor containing 1 g of catalyst under stirring at 700 rpm. A glass electrode for measuring the pH of the reactional medium was connected to the reactor. Similarly, it was connected to a burette containing a 1 M solution of NaOH enabling it to be dropped in by manual action. The pH in the reactor was adjusted to 8.0 by means of the addition of a sufficient quantity of the NaOH solution and maintained for the duration of the reaction. A flow of 30 mLmin⁻¹ of air was let in through the bubbler. A reaction temperature of 60°C was maintained. After 6 hours of reaction in these conditions, a total conversion of 1,2 propanediol and the following distribution of selectivities between lactic acid, pyruvic acid and acetol were obtained: 61%, 27% and 12%, respectively.

## Claims

1. An oxidative catalytic process for the synthesis of lactic acid, by oxidation of the OH group of the primary carbon of 1,2-propanediol, carried out in a reactor from 1,2-propanediol and a gaseous stream containing oxygen, in an alkaline aqueous medium, in the presence of a catalyst containing a noble metal, **characterised in that** it comprises the following stages:
1) activation of the catalyst: reduction of the noble metal by means of contact between the catalyst and a gaseous stream containing hydrogen;
2) supply of the reactor: providing the reactor with the reduced catalyst and an aqueous solution of 1-2-propanediol;
3) oxidation reaction: bubbling a gaseous stream containing oxygen by means of a mixture of the catalyst and the solution of 1,2-propanediol maintaining the pH at a fixed value, by means of the addition of an alkaline solution to the reaction, and controlling the heating, the stirring and the internal pressure of the reactor;
4) separation of the products: removal of the catalyst by filtration and separation of the lactic acid of the aqueous phase.

2. Oxidative catalytic process for the synthesis of lactic acid, according to claim 1, **characterised in that** said catalyst comprises a metal oxide support, with a specific surface area in the range between 50 m²g⁻¹ and 1000 m²g⁻¹, selected from Al₂O₃, TiO₂, SiO₂ and ZrO₂, Nb₂O₅, CeO₂, MgO, ZSM-5, MCM-22, MCM-41 and aluminium silicates with a zeolitic structure, preferably Al₂O₃, SiO₂, TiO₂ or ZrO₂, and a noble metal selected from Pt, Pd, Ru, Rh and Ir, or a combination of these, preferably Pt and Pd, but preferably a Pt/Al₂O₃ catalyst.

3. Oxidative catalytic process for the synthesis of lactic acid, in accordance with claims 1 and 2, **characterised in that** said catalyst has a noble metal content between 0.01 % and 10% p/p, preferably between 0.1 % and 5% p/p.

4. Oxidative catalytic process for the synthesis of lactic acid, according to claims 1, 2 and 3, **characterised in that** said activation of the catalyst is previously carried out "in situ" in the reactor or "ex situ", by means of contact between the catalyst and a gas current containing a hydrogen content in the range between 0.5% and 100% p/p, preferably between 10% and 100%, at the flow rate in the range between 0.5 mLmin⁻¹ and 200 mLmin⁻¹, preferably between 1 mLmin⁻¹ and 150 mLmin⁻¹, at the temperature in the range between 200°C and 500°C, preferably between 300°C and 500°C, for a period in the range between 0.5 and 5 hours, preferably between 1 and 5 hours.

5. Oxidative catalytic process for the synthesis of lactic acid, according to claim 1, **characterised in that** said supply of the reactor is carried out in batches or in a continuous manner.

6. Oxidative catalytic process for the synthesis of lactic acid, according to claim 1, **characterised in that** said oxidation reaction of the OH group of the primary carbon is carried out under the following reaction conditions: concentration of the 1,2-propanediol solution in the range between 0.01 M up to the pure substrate, catalyst/1,2-propanediol solution ratio in the range between 1/4 and 1/20, pH of the fixed reaction in the value in the range between 7 and 14.0, preferably between 8.0 and 12.0, by means of the controlled addition of an alkaline solution, selected from solutions of hydroxides and carbonates of alkali metals and alkali earth metals, preferably NaOH or KOH, with a concentration in the range between 0.1 M and 2 M, preferably in the range between 0.5 M and 1.5 M, stirring in the range between 200 rpm and 2000 rpm, preferably between 600 rpm and 1200 rpm, temperature in the range between 20°C and 100°C, but preferably between 30°C and 70°C, pressure in the range between 1 bar and 5 bar, preferably between 1 bar and 3 bar, gaseous air stream containing oxygen in nitrogen with a concentration in the range between 0.5% and 100% v/v, preferably between 10% and 30%, introduced in the reactor at a flow in the range between 10 mLmin⁻¹ and 100 mLmin⁻¹.
